# EUROPÄISCHE PATENTSCHRIFT

(11) **EP 2 091 596 B1**
(45) Veröffentlichungstag und Bekanntmachung des Hinweises auf die Patenterteilung: **16.03.2011**
(21) Anmeldenummer: 06841150.3
(22) Anmeldetag: 22.12.2006
(51) Int. Cl.: A61M 5/145, G01L 7/00

(54) **VORRICHTUNG UND VERFAHREN ZUR KONTROLLE UND ÜBERWACHUNG DES DRUCKES IN DRUCKLEITUNGEN BZW. ROHREN**
DEVICE AND METHOD FOR CHECKING AND MONITORING THE PRESSURE IN PRESSURE PIPES AND/OR CONDUITS
DISPOSITIF ET PROCÉDÉ DE CONTRÔLE ET DE SURVEILLANCE DE LA PRESSION DANS DES CONDUITES DE PRESSION OU DES TUYAUX

(43) Veröffentlichungstag der Anmeldung: 26.08.2009
(73) Patentinhaber: Fraunhofer-Gesellschaft zur Förderung der angewandten Forschung e.V., 80686 München (DE)
(72) Erfinder: TRIEU, Hoc, Khiem, 47475 Kamp-Lintfort (DE); WIEBE, Peter, 40882 Ratingen (DE); KLIEBER, Robert, 44379 Dortmund (DE)
(74) Vertreter: Schenk, Markus
(86) Internationale Anmeldenummer: PCT/EP2006/012513
(87) Internationale Veröffentlichungsnummer: WO 2008/083692

(56) Entgegenhaltungen:
- WO-A-00/72900
- WO-A-2005/102416
- WO-A-2006/116997
- WO-A2-01/72357
- US-A- 5 808 203

## Beschreibung

Die vorliegende Erfindung bezieht sich auf eine Vorrichtung und ein Verfahren, die insbesondere geeignet sind, einen Druck in Druckleitungen bzw. Rohren zu messen, zu überwachen und zu kontrollieren.

In biologischen oder medizinischen Anwendungen werden oft Spritzen verwendet, um beispielsweise Flüssigkeiten in (Druck-) Leitungen oder Rohren einzubringen. Bei Verwendung einer Spritze ist in Abhängigkeit des Druckes in den Druckleitungen oder Rohren eine mehr oder weiniger große Kraft für das Einbringen einer beispielhaften Flüssigkeit oder Gases erforderlich. Der Druck in diesen Leitungen wird normalerweise dadurch gemessen, dass direkt an den Rohrleitungen ein Sensor zur Messung des Druckes, d.h. ein Drucksensor, angebracht wird. Diese Verfahrensweise kann sich jedoch als schwierig erweisen, da es dazu erforderlich ist, den Sensor in die Rohrleitung zu integrieren oder, sofern dies nicht möglich ist, einen komplizierten Aufbau zu finden, um den Sensor mit der Leitung bzw. deren Innenraum in Verbindung zu bringen. Dies wäre möglich, wenn die Leitung elastisch ist und eine Druckmessung des Innendruckes der Leitung beispielsweise mittels einer Messung der Spannung der elastischen Leitung erfolgen kann, z.B. durch eine Messung einer Kraft, die erforderlich ist, um die elastische Leitung zu verformen (z.B. um die Leitung durchzudrücken).

Wenn jedoch die Leitung nicht elastisch ist, wie dies z.B. bei einer Leitung mit einem sehr dünnen Durchmesser der Fall ist, ist es schwierig, diese Messtechnik auszuführen. Falls dies nicht möglich oder zu ungenau ist, müsste die Leitung ein Loch aufweisen, um dadurch den Innendruck direkt messen zu können. Dies wiederum kann zu einem Druckverlust bzw. zu einem Leck führen.

Eine aus dem Stand der Technik bekannte Vorrichtung ist im Dokument WO 01/72357 A2 offenbart. Der Oberbegriff der Ansprüche 1 und 8 ist auf diesem Dokument basiert.

Es ist die Aufgabe der vorliegenden Erfindung eine Vorrichtung und ein Verfahren zu schaffen, die es ermöglichen, einen Druck innerhalb von Leitungen und Rohren flexibel zu messen und zu kontrollieren.

Diese Aufgabe wird durch eine Vorrichtung gemäß Anspruch 1 und ein Verfahren gemäß Anspruch 8 gelöst.

Der vorliegenden Erfindung liegt die Erkenntnis zugrunde, dass der Druck eines Messmediums innerhalb einer Leitung dadurch gemessen werden kann, dass das Messmedium mit einer Kammer über eine Kammeröffnung verbunden wird und die Kammer ferner einen beweglichen Kolben aufweist, wobei eine Bewegung des Kolbens zu einer Veränderung eines Kammervolumen führt. Nach Druckausgleich zwischen einem Medium in der Kammer und dem Messmedium in der Leitung kann der Druck des Messmediums in der Leitung durch eine Druckmessung in der Kammer erfolgen. Alternativ dazu kann eine Messung einer Kraft, die auf dem Kolben einwirkt, vorgenommen werden. Die Kammer mit dem Kolben kann dabei beispielsweise eine Spritze sein, wobei erfindungsgemäß entweder die Spritze eine Einrichtung zum Messen des Druckes oder zum Messen der Kraft aufweist oder aber die Spritze über einen optionalen Adapter mit einer Einrichtung zum Messen einer Kraft bzw. eines Druckes verbunden ist.

Dies ist insbesondere dann vorteilhaft, wenn es unabhängig von der Druckmessung ohnehin erforderlich ist, eine Spritze zu verwenden, um beispielsweise eine Flüssigkeit in die Leitung einzubringen. In einem solchen Fall kann gleichzeitig die Kraft gemessen werden, die nach einem Einbringen der Spritzennadel in die Leitung auf die Spritze auszuüben ist, um beispielsweise dem Druck in der Leitung standzuhalten oder die Kraft zu ermitteln, die erforderlich ist, um beispielsweise ein bestimmtes Volumen des Mediums in einer gewissen Zeit in die Leitung einzubringen. Somit kann die Spritze sowohl als Injektionsgerät als auch als Vorrichtung zur Kraft- bzw. Druckmessung verwendet werden.

Das erfindungsgemäße Konzept ist aber nicht nur auf Leitungen mit Flüssigkeiten und deren Druckmessung beschränkt, sondern kann in gleicher Weise für Leitungen, die gasförmige Stoffe enthalten, angewendet werden.

Erfindungsgemäß wird somit der Druck in der Leitung bzw. in einem Rohr oder in der Spritze selbst dadurch gemessen, dass eine Kraftmessung vorgenommen wird, wobei die gemessene Kraft der Kraft entspricht, die auf einen Kolben der Spritze auszuüben ist, so dass das Medium in der Spritze in die Leitung gelangt. Andererseits kann mittels eines Drucksensors, der im direkten Kontakt mit dem Spritzeninhalt steht, der Druck direkt bestimmt werden. Da der Druck ein Maß für die Kraft auf eine Flächeneinheit ist, kann durch eine Messung der Kraft und des Flächeninhalts A (der Querschnittsfläche) der Druck beispielsweise innerhalb der Leitung berechnet werden. Wenn beispielsweise die Spritze einen kreisförmigen Querschnitt mit einem Radius r aufweist, dann kann der Druck P mit Hilfe der Kraft F, die auf den Kolben der Spritze einwirkt, durch P=F/A berechnet werden, wobei A=πr² Die Kraft F in dieser Formel ist dabei jene Kraft, die erforderlich ist, um den Kolben in einer fixierten Position zu halten, nachdem ein Druckausgleich zwischen der Kammer der Spritze und dem Innendruck der Leitung hergestellt wurde.

Die vorliegende Erfindung weist somit die folgenden wichtigen Aspekte auf: Die Druckmessung innerhalb einer Druckleitung oder einem Rohr oder einer Spritze wird mittels eines Sensors für die Kraft oder den Druck, der beispielsweise auf die Spritze einwirkt, vorgenommen, wobei die Spritze mit der Leitung oder dem Rohr verbunden ist.

Der beispielhafte Drucksensor kann beispielsweise in dem Gehäuse der Spritze integriert sein und kann möglicherweise ein Druckwandelmedium aufweisen, welches, sofern erforderlich, eine Membran zum Trennen des Druckwandelmediums bzw. des Drucksensors von der Umgebung aufweist. Andererseits kann der Drucksensor auch in einem Gehäuse untergebracht sein, welches mit dem Kolben der Spritze verbunden ist und durch ein Drücken der Spritze einen Druck im Drucksensor erzeugt. Dadurch kann durch eine Messung des Druckes in dem Drucksensor der Druck in der Spritze und/oder der Druck in der Leitung oder des Rohres bestimmt werden. Ein Druckwandelmedium ist beispielsweise flüssig oder gasförmig und wandelt eine Kraft, die beispielsweise auf dem Kolben wirkt, in einen Druck des Druckwandelmediums um bzw. leitet diesen Druck an den Sensor weiter. Vorzugsweise sollte es nicht stark komprimierbar sein, um eine unmittelbare Weiterleitung zu ermöglichen.

Andererseits kann ein Kraftsensor dazu verwendet werden, die Kraft, die auf die Spritze ausgeübt wird, um den Druck innerhalb der Leitung standhalten zu können, direkt zu messen bzw. die Kraft, die wenigstens erforderlich ist, um das Medium innerhalb der Spritze in die Leitung einzubringen. Unter Verwendung der Querschnittsfläche A der Spritze bzw. des Flächeninhalts des Kolbens der Spritze kann somit der Druck innerhalb der Spritze und dadurch schließlich der Druck innerhalb der Leitung wie oben beschrieben berechnet werden.

Es ist darüber hinaus auch möglich beide Aspekte in einen modularen Setup unter Verwendung eines Adapters zu kombinieren. Der Adapter ist dabei ausgelegt, um eine Verbindung zwischen der gewählten Spritze und dem Druck- bzw. Kraftsensor herzustellen. Vorzugsweise ist der Adapter derart ausgelegt, dass er auf eine konventionelle Spritze bzw. auf einen Kolben einer konventionellen Spritze passt und andererseits an den Druck- bzw. Kraftsensor koppelbar ist. Unter Verwendung verschiedener Adapter, die an die Kolben der verschiedenen Spritzen angepasst sind, kann schließlich jede beliebige konventionelle Spritze in Kombination mit dem (universellen) Druck- bzw. Kraftsensors zur Druckmessung verwendet werden.

Bevorzugte Ausführungsbeispiele der vorliegenden Erfindung werden nachfolgend Bezug nehmend auf die beiliegenden Zeichnungen näher erläutert. Es zeigen:
- Fig. 1: eine Vorrichtung mit einer variablen Kammer und einer Einrichtung zum Messen des Druckes; und
- Fig. 2: einen Drucksensor der mit einem Adapter mit einer Spritze verbunden ist.

Bevor im Vorliegenden die vorliegende Erfindung anhand der Zeichnungen näher erläutert wird, wird darauf hingewiesen, dass gleiche oder gleichwirkende Elemente in den Figuren mit den gleichen oder ähnlichen Bezugszeichen versehen sind, und dass eine wiederholte Beschreibung dieser Elemente weggelassen wird.

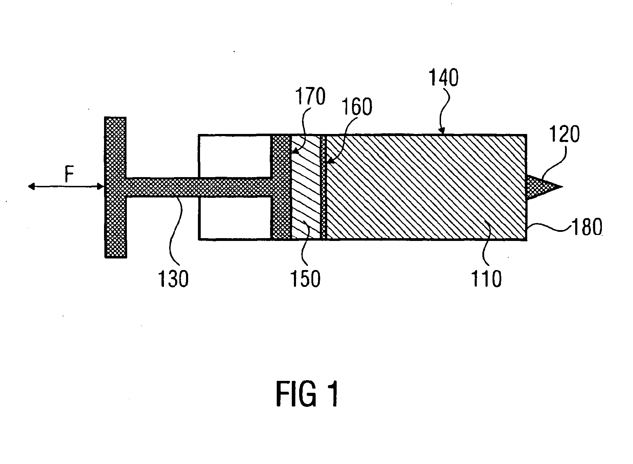

Fig. 1 zeigt eine Vorrichtung mit einer Kammer 110 mit einer Kammeröffnung 120 und einem Kolben 130, wobei der Kolben 130 ein Kammervolumen, welches durch eine Kammerwand 140, den Kolben 130 einerseits und einem Boden 180 mit der Kammeröffnung 120 andererseits begrenzt ist, gebildet ist. Außerdem weist die Vorrichtung eine Einrichtung 150 zum Messen eines Druckes in der Kammer 110 oder zum Messen einer Kraft, die auf den Kolben 130 wirkt, um das Kammervolumen zu verändern, auf.

Die Einrichtung 150 weist dabei eine Sensorfläche 160 auf, welche in dem hier gewählten Ausführungsbeispiel mit einer Querschnittsfläche 170 senkrecht zur Bewegungsrichtung des Kolbens 130 identisch ist. Bei anderen Ausführungsbeispielen erstreckt sich die Sensorfläche 160 nur über einen Teilbereich der Querschnittsfläche 170. Mit Hilfe des Flächeninhaltes A der Sensorfläche 160 und die auf den Kolben 130 einwirkende Kraft F, um das Volumen der Kammer 110 zu verändern, kann der Druck P wie folgt bestimmt werden: P=F/A. Eine Zugkraft (z.B. ein negatives Vorzeichen der Kraft F) entspricht dabei einem Unterdruck in der Leitung, währenddessen ein Überdruck beispielsweise einem positivem Vorzeichen der Kraft F entspricht.

Bei weiteren Ausführungsbeispielen ist die Einrichtung 150 an einer anderen Stelle angeordnet. Beispielsweise kann die Einrichtung 150 entlang eines Seitenabschnittes der Wand 140 angeordnet oder aber, sofern sie über eine entsprechende Öffnung verfügt, auch an dem Boden 180 mit der Öffnung 120 angeordnet sein. Die Einrichtung 150 kann weiterhin entweder eine Anzeige aufweisen, die den Druck bzw. die auf sie einwirkende Kraft anzeigt, oder aber die Einrichtung 150 ist drahtlos oder mit Leitungen an einer Auswerteelektronik angeschlossen, die in der vorliegenden Zeichnung nicht gezeigt ist.

Bei einem weiteren Ausführungsbeispiel kann der Druck innerhalb der Kammer 110 dadurch bestimmt werden, dass auf dem Kolben 130 eine konstante Kraft einwirkt und eine Zeit gemessen wird, in der ein bestimmtes Volumen durch die Kammeröffnung 120 in die Leitung (in der Figur nicht gezeigt) eingebracht ist.

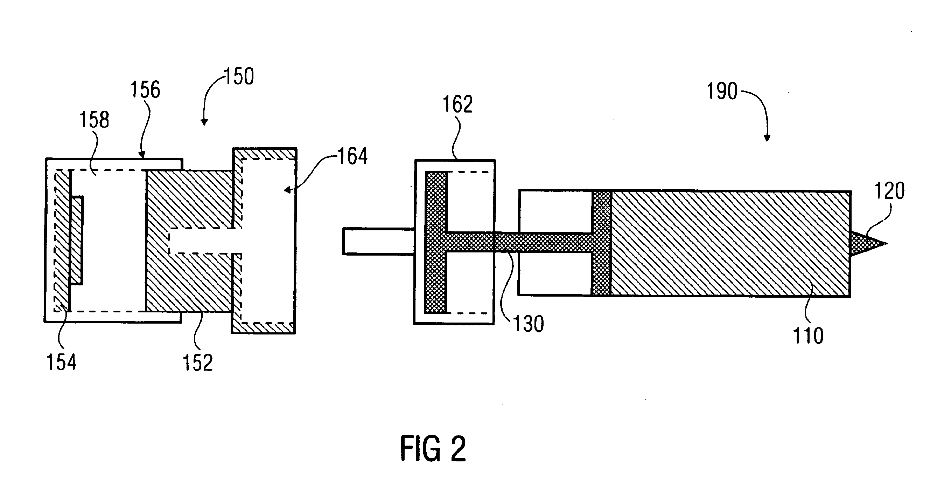

Fig. 2 zeigt eine Einrichtung 150 zur Messung des Drucks bzw. der Kraft, die mit einem Adapter für eine Spritze 190 verbunden ist. Die Spritze 190 umfasst die Kammer 110 mit der Kammeröffnung 120 und dem Kolben 130, der wiederum beweglich ist und durch die Bewegung das Volumen der Kammer 110 verändert. Bei diesem Ausführungsbeispiel wird ein Drucksensor dazu benutzt, um eine Kraft zu messen, die erforderlich ist, um die Spritze 190 zu drücken. Die Einrichtung 150 weist dabei einen Stempel 152, einen Sensor 154, der an einem Gehäuse 156 mit einem Druckumwandlungsmedium 158 angeordnet ist, auf. Diese modulare Anordnung weist weiterhin einen Adapter 162 auf, der derart ausgelegt ist, dass der Kolben 130 einerseits und andererseits der Adapter 162 an eine Öffnung 164 der Einrichtung 150 passt. Vorzugweise wird der Adapter 162 für eine gegebene Spritze 190 angepasst, d.h. unter Verwendung verschiedener Adapter, die sich hinsichtlich ihrer Öffnung für die Kolben 130 unterscheiden, kann die Einrichtung 150 für verschiedene Spritzen 190 verwendet werden. Somit kann eine einfache Verbindung für jede verfügbare Spritze 190 mit der Einrichtung 150 zum Messen des Druckes oder zum Messen der Kraft hergestellt werden. Die Einrichtung 150 ist somit universell einsetzbar.

Es ist ein Vorteil der vorliegenden Erfindung, dass es mit Hilfe des Adapters 162 und 150 möglich ist, den Druck in Leitungen oder Rohren oder Spritzen in einer kontrollierten Art und Weise nicht nur zu messen bzw. zu berechnen, sondern eine Überlastung der Leitung infolge eines zu hohen Druckes zu vermeiden. Ein Überschreiten eines kritischen Grenzwertes kann beispielsweise durch ein optisches oder akustisches Signal erfolgen. Alternative kann auch ein elektrisches Signal beispielsweise an eine Auswerteelektronik (nicht gezeigt) erfolgen. Die Druckmessung in dem Sensor 154 kann beispielsweise mittels verschiedener Piezo- oder Dünnfilmsensoren oder mittels einer Widerstandsmessung in einer Keramikmesszelle vorgenommen werden.

Zusammenfassend bietet die vorliegende Erfindung somit eine einfache und kostengünstige Möglichkeit, unter Verwendung konventioneller Spritzen 190 den Druck innerhalb von Leitungen zu bestimmen. Ferner kann die vorliegende Erfindung als Schutz benutzt werden, mit deren Hilfe eine zu hohe Beanspruchung der Leitung durch Erzeugen eines zu hohen Druckes in der Leitung beispielsweise während des Injizierens eines Mediums, vermieden wird.

Die vorliegende Erfindung ist somit insbesondere dann vorteilhaft, wenn für eine vorliegende Anwendung ohnehin eine Spritze 190 beispielsweise zum Einbringen oder Entnehmen einer Flüssigkeit oder von Gas in eine Leitung oder Rohr verwendet wird. In diesem Fall können die verwendeten Spritzen 190 und unter Verwendung des Stempels 152 oder des Adapters 162 zur Messung des Druckes verwendet werden. Erfindungsgemäß kann damit sowohl der Druck gemessen werden, der in einem Vergleich zu dem Umgebungsdruck größer ist als auch ein Unterdruck in den Leitungen gemessen werden. Ein Unterdruck macht sich dabei derart bemerkbar, dass die auf den Kolben 130 einwirkende Kraft F bzw. die Kraft F, die erforderlich ist, um den Kolben 130 in einer gegebenen Stellung zu fixieren, als eine Zugkraft in Richtung der Kammeröffnung 120 wirkt. Andererseits ist eine Druckkraft F, die auf den Kolben 130 wirkt, eine Kraft, die von der Kammeröffnung 120 weggerichtet ist.

## Patentansprüche

1. Vorrichtung mit:
einer Kammer (110) mit mindestens einer Kammeröffnung (120);
einem Kolben (130), der in der Kammer (110) beweglich ist, um ein durch den Kolben (130) und einer Kammerwand (140) definiertes Kammervolumen, das an die Kammeröffnung (120) angrenzt, zu verändern; und
einer Einrichtung (150) zum Messen einer Kraft F, die auf den Kolben (130) wirkt, um das Kammervolumen zu verändern,
**dadurch gekennzeichnet, dass**
die Einrichtung(150) einen Stempel (152) und einen Druckbehälter (156) aufweist, so dass eine Bewegung des Stempels (152) ein Volumen oder einen Druck des Druckbehälters (156) verändert und der Stempel (152) mit dem Kolben (130) koppelbar ist, so dass das Volumen des Druckbehälters (156) sich entsprechend des Druckes in der Kammer (110) verändert, wobei die Einrichtung (150) mittels eines Adapters (162) mit dem Kolben (130) koppelbar ist.

2. Vorrichtung gemäß Anspruch 1, bei der der Druckbehälter (156) ein Druckwandlungsmedium aufweist.

3. Vorrichtung gemäß einem der vorhergehenden Ansprüche, bei der die Einrichtung (150) zum Messen der Kraft ausgelegt ist, die auf dem Kolben (130) in Richtung der Kammeröffnung (120) einwirkt, zu messen.

4. Vorrichtung gemäß einem der vorhergehenden Ansprüche, bei der die Kammeröffnung (120) eine Kanüle aufweist.

5. Vorrichtung gemäß einem der vorhergehenden Ansprüche, bei der die Kammer (110) ein weiteres Druckwandlungsmedium aufweist.

6. Vorrichtung gemäß einem der vorhergehenden Ansprüche, bei der die Einrichtung (150) ausgelegt ist, um bei Erreichen eines oberen Grenzwertes des Druckes in der Kammer (110) ein beliebiges Signal, beispielsweise ein optisches, akustisches oder elektrisches Signal zu geben.

7. Vorrichtung gemäß Anspruch 5, bei dem das Druckwandlungsmedium durch eine Membran von einer Umgebung getrennt ist.

8. Verfahren mit:
Bereitstellen einer Kammer (110) mit mindestens einer Kammeröffnung (120), einen Kolben (130), der in der Kammer (110) beweglich ist und ein Kammervolumen definiert, wobei das Kammervolumen an die Kammeröffnung (120) angrenzt und durch den Kolben (130) und einer Kammerwand (140) gegeben ist; und
Messen einer Kraft, die auf dem Kolben (130) einwirkt,
**dadurch gekennzeichnet, dass**
der Schritt des Messens der Kraft derart ausgeführt wird, dass der Kolben (130) mit einem Stempel (152), der ein Volumen eines Druckbehälters (156) definiert, verbunden wird und das Messen der Kraft durch ein Messen eines Druckes, der in dem Druckbehälter (156) durch eine Bewegung des Stempels (152) entsteht, ausgeführt wird, wobei der Schritt des Messens der Kraft ein Verwenden eines Adapters (162) umfasst, wobei der Adapter (162) eine Kopplung zwischen dem Kolben (130) und dem Stempel (152) herstellt.

9. Verfahren gemäß Anspruch 8, bei dem der Schritt des Messens des Druckes in der Kammer (110) ein Messen eines Unterdruckes aufweist, und der Unterdruck durch ein Messen einer Zugkraft des Kolbens (130), die zur Kammeröffnung (120) wirkt, gemessen wird.

10. Verfahren gemäß Anspruch 8 oder Anspruch 9 , bei dem der Schritt des Bereitstellens einer Kammer (110) mit einer Kammeröffnung (120) ein Bereitstellen einer Spritze umfasst und die Kammeröffnung (120) eine Kanüle aufweist.

11. Verfahren gemäß einem der Ansprüche 8 bis 10, bei dem die Kammer (110) mit der Kammeröffnung (120) in Verbindung gebracht wird mit einem Messmedium in einer Leitung und bei dem der Schritt des Messens des Druckes oder des Messens der Kraft eine Verwendung eines Messmediums in der Kammer (110) umfasst.

12. Verfahren gemäß einem der Ansprüche 8 bis 11, bei dem der Schritt des Messens des Druckes oder des Messens der Kraft eine Verwendung eines Druckwandelmediums in dem Druckbehälter (156) und/oder in der Kammer (110) umfasst.

13. Verfahren gemäß einem der Ansprüche 8 bis 12, bei dem der Schritt des Messens des Druckes oder des Messens der Kraft ein Signalisieren eines Erreichens einer Maximalkraft oder eines Maximaldruckes umfasst.

## Claims

1. An apparatus comprising:
a chamber (110) comprising at least one chamber opening (120);
a plunger (130) which is movable within the chamber (110) so as to change a chamber volume which is defined by the plunger (130) and a chamber wall (140), and which adjoins the chamber opening (120); and
a means (150) for measuring a force F which acts upon the plunger (130) so as to change the chamber volume,
**characterized in that**
the means (150) comprises a stamp (152) and a pressure vessel (156), so that a motion of the stamp (152) changes a volume or a pressure of the pressure vessel (156), and the stamp (152) is couplable to the plunger (130), so that the volume of the pressure vessel (156) will change in accordance with the pressure within the chamber (110), and wherein the means (150) may be coupled to the plunger (130) by means of an adapter (162).

2. The apparatus as claimed in claim 1, wherein the pressure vessel (156) comprises a pressure transducer medium.

3. The apparatus as claimed in any of the previous claims, wherein the means (150) is adapted to measure the force which acts upon the plunger (130) in the direction of the chamber opening (120).

4. The apparatus as claimed in any of the previous claims, wherein the chamber opening (120) comprises a drain tube.

5. The apparatus as claimed in any of the previous claims, wherein the chamber (110) comprises a further pressure transducer medium.

6. The apparatus as claimed in any of the previous claims, wherein the means (150) is implemented to emit a random signal, for example an optical, acoustic or electrical signal, when an upper threshold value of the pressure within the chamber (110) is reached.

7. The apparatus as claimed in claim 5, wherein the pressure transducer medium is separated from a surrounding by a membrane.

8. A method comprising:
providing a chamber (110) comprising at least one chamber opening (120), a plunger (130) which is movable within the chamber (110) and defines a chamber volume, the chamber volume adjoining the chamber opening (120) and being defined by the plunger (130) and a chamber wall (140); and
measuring a force acting upon the plunger (130),
**characterized in that**
the step of measuring the force is performed such that the plunger (130) is connected to a stamp (152) which defines a volume of a pressure vessel (156), and that measuring the force is performed by measuring a pressure which arises within the pressure vessel (156) by a motion of the stamp (152), and wherein the step of measuring the force comprises utilizing an adapter (162), the adapter (162) establishing a coupling between the plunger (130) and the stamp (152).

9. The method as claimed in claim 8, wherein the step of measuring the pressure within the chamber (110) comprises measuring a negative pressure, and wherein the negative pressure is measured by measuring a tractive force of the plunger (130) which acts toward the chamber opening (120).

10. The method as claimed in claims 8 or 9, wherein the step of providing a chamber (110) comprising a chamber opening (120) comprises providing a syringe, and wherein the chamber opening (120) comprises a drain tube.

11. The method as claimed in any of claims 8 to 10, wherein the chamber (110) comprising the chamber opening (120) is connected to a measurement medium within a line, and wherein the step of measuring the pressure or measuring the force comprises using a measurement medium within the chamber (110).

12. The method as claimed in any of claims 8 to 11, wherein the step of measuring the pressure or measuring the force comprises using a pressure transducer medium within the pressure vessel (156) and/or within the chamber (110).

13. The method as claimed in any of claims 8 to 12, wherein the step of measuring the pressure or measuring the force comprises signaling that a maximum force or a maximum pressure has been reached.

## Revendications

1. Dispositif avec:
une chambre (110) avec au moins une ouverture de chambre (120);
un piston (130) mobile dans la chambre (110), pour modifier un volume de chambre, défini par le piston (130) et une paroi de chambre (140), qui est adjacent à l'ouverture de chambre (120); et
un moyen (150) pour mesurer une force F agissant sur le piston (130) pour modifier le volume de chambre,
**caractérisé par le fait que**
le moyen (150) présente un poinçon (152) et un réservoir à pression (156), de sorte qu'un mouvement du poinçon (152) modifie un volume ou une pression d'un réservoir à pression (156) et que le poinçon (152) peut être couplé au piston (130), de sorte que le volume du réservoir à pression (156) varie selon la pression dans la chambre (110), le moyen (150) pouvant être couplé au piston (130) à l'aide d'un adaptateur (162).

2. Dispositif selon la revendication 1, dans lequel le réservoir à pression (156) présente un fluide de conversion de pression.

3. Dispositif selon l'une des revendications précédentes, dans lequel le moyen (150) pour mesurer la force est conçu pour mesurer la force agissant sur le piston (130) en direction de l'ouverture de chambre (120).

4. Dispositif selon l'une des revendications précédentes, dans lequel l'ouverture de chambre (120) présente une canule.

5. Dispositif selon l'une des revendications précédentes, dans lequel la chambre (110) présente un autre fluide de conversion de pression.

6. Dispositif selon l'une des revendications précédentes, dans lequel le moyen (150) est conçu pour donner, en cas d'atteinte d'une valeur limite supérieure de la pression dans la chambre (110), un signal quelconque, par exemple un signal optique, acoustique ou électrique.

7. Dispositif selon la revendication 5, dans lequel le fluide de conversion de pression est séparé d'un environnement par une membrane.

8. Procédé avec:
préparer une chambre (110) avec au moins une ouverture de chambre (120), un piston (130) mobile dans la chambre (110) et définissant un volume de chambre, le volume de chambre étant adjacent à l'ouverture de chambre (120) et étant déterminé par le piston (130) et une paroi de chambre (140); et
mesurer une force agissant sur le piston (130),
**caractérisé par le fait que**
l'étape de mesure de la force est exécutée de sorte que le piston (130) soit relié à un poinçon (152) définissant un volume d'un réservoir à pression (156) et que la mesure de la force est effectuée par une mesure d'une pression qui se produit dans le réservoir à pression (156) par un mouvement du poinçon (152), l'étape de mesure de la force comprenant une utilisation d'un adaptateur (162), l'adaptateur (162) réalisant un couplage entre le piston (130) et le poinçon (152).

9. Procédé selon la revendication 8, dans lequel l'étape de mesure de la pression dans la chambre (110) présente une mesure d'une dépression, et la dépression est mesurée par une mesure d'une force de traction du piston (130), agissant vers l'ouverture de chambre (120).

10. Procédé selon la revendication 8 ou la revendication 9, dans lequel l'étape de préparation d'une chambre (110) avec une ouverture de chambre (120) présente une préparation d'une seringue et l'ouverture de chambre (120) présente une canule.

11. Procédé selon l'une des revendications 8 à 10, dans lequel la chambre (110) avec l'ouverture de chambre (120) est amenée en communication avec un fluide de mesure dans un conduit et dans lequel l'étape de mesure de la pression ou de mesure de la force comprend une utilisation d'un fluide de mesure dans la chambre (110).

12. Procédé selon l'une des revendications 8 à 11, dans lequel l'étape de mesure de la pression ou de mesure de la force comprend une utilisation d'un fluide de conversion de pression dans le réservoir à pression (156) et/ou dans la chambre (110).

13. Procédé selon l'une des revendications 8 à 12, dans lequel l'étape de mesure de la pression ou de mesure de la force comprend une signalisation de l'atteinte d'une force maximale ou d'une pression maximale.
